Europäisches Patentamt

European Patent Office

Office européen des brevets

(19)

(11) Publication number : **0 391 318 B1**

(12) **EUROPEAN PATENT SPECIFICATION**

(45) Date of publication of patent specification :
07.07.93 Bulletin 93/27

(51) Int. Cl.⁵ : **A23L 2/26,** A23L 2/36,
A23L 3/34, A61N 1/44,
C02F 1/28

(21) Application number : 90106306.5

(22) Date of filing : 02.04.90

(54) **Ion water for production of foods and beverages.**

(30) Priority : 03.04.89 JP 84534/89
17.08.89 JP 211885/89

(43) Date of publication of application :
10.10.90 Bulletin 90/41

(45) Publication of the grant of the patent :
07.07.93 Bulletin 93/27

(84) Designated Contracting States :
AT BE CH DE DK ES FR GB GR IT LI LU NL SE

(56) References cited :
EP-A- 0 195 819
EP-A- 0 227 174
DE-A- 2 705 433

(56) References cited :
PATENT ABSTRACTS OF JAPAN, vol. 10, no.4
(C-322)(2001), 9 Jan 1986; & Jp-A 60164467
(Rooman Rogyo K.K),27.08.1985
PATENT ABSTRACTS OF JAPAN,
vol,10,no.43(C-329)(2100) 20 February 1986; &
JP-A- 60190718 (SHINPEI MAJIMA) 28.09.1985

(73) Proprietor : **Nasu, Atsushi**
**99 Katako**
**Youkaichiba-shi Chiba-ken (JP)**

(72) Inventor : **Nasu, Atsushi**
**99 Katako**
**Youkaichiba-shi Chiba-ken (JP)**

(74) Representative : **Popp, Eugen, Dr. et al**
**MEISSNER, BOLTE & PARTNER**
**Widenmayerstrasse 48 Postfach 86 06 24**
**W-8000 München 86 (DE)**

## Description

BACKGROUND OF THE INVENTION

1. Field of the Invention:

The present invention relates to ion water for use in the production of beverages and foods, and more specifically, it relates to ion water utilizing salt separated from sea water.

2. Related Art Statement:

Heretofor, the production of bread and noodles has been characterised by the addition of large quantities of salt. The reasons for the addition of salt are as follows:

(1) Gluten contained in flour etc. imparts tackiness and elasticity when it absorbs water and swells. When the swollen gluten encounters inorganic matter a constriction action takes place and it becomes firm and solid. In other words, salt helps to make noodles with a so-called 'strong body'.

(2) It prevents the generation of cracks otherwise liable to occur during drying.

(3) It inhibits enzymatic activity and can therefore prevent a gradual reduction on elasticity.

(4) It has a bacteriostatic action.

(5) It imparts a salty taste.

When making bread, salt is invariably one of the ingredients. Bread is produced by kneading flour with salt, sugar, edible oil, water etc., allowing the resulting dough to stand and rise with carbon dioxide, and then baking the risen dough. The role of the salt is mainly to improve the glutinousness of the dough enabling the carbon dioxide gas to swell thin films of gluten to produce voluminous, tasty bread. Further, other roles of the added salt are to control the fermentation of the yeast and to impart a salty taste enhancing the flavour of the bread.

However, overdoses of salt can cause various diseases, such as hypertension, heart disease, cerebral haemorrhage etc., and it is advisable that the daily intake of salt should not be more than 5g.

Since noodles contain a considerable amount of salt, it is not desirable to eat a great quantity of noodles when a reduced salt intake is required.

Further, other additives include salt water and are considerably undesirable for health reasons and it is better if their use could be avoided.

Returning to the prior art processes used for the production of noodles, if salt or salt water is not used, it is impossible to produce noodles which retain a good taste and mouthfeel for the predetermined time and still do not go bad.

This also applies to bread. The production of bread requires the use of salt in such quantities as 1 - 3% by weight based on the flour content.

Furthermore, in a different application, water stored for emergency use or water which is canned or bottled is expensive and requires considerable storage space. The ion water of the present invention can mitigate these disadvantages.

The invention can also assist in supplying calcium. If calcium is insufficient in the diet not only are bones and teeth weakened but also various diseases such as kidney disease are brought about.

The tendency to eat too much acidic foods is also believed to cause various diseases and it is well recognised that more alkaline foods are good for the health.

Furthermore, the role of silicon in vivo, in particular its influence exerted on the metabolism of mineral elements such as phosphorus, has been attracting attention and it has been reported that with those living in regions where the silicon content in drinking water is high have an increased blood calcium content over the normal levels.

Silicon is abundant in nature as quartz minerals which are utilized in various fields, but silicon in the hydrosphere which is present in sea water as orthosilicic acid ions or monosilicic acid ions has hitherto been hardly utilized.

SUMMARY OF THE INVENTION

The present inventors have been intensively studying the separation and utilization of various elements contained in sea water, and have discovered that salt containing considerable amounts of potassium, magnesium, silicon etc. may be separated by the prescribed method (Japanese patent Application No. 201578/1987), and by utilizing such a salt, the aim has been to solve the problems involved in the above-described prior art food additives and stored water etc. Accordingly, an object of the present invention is to provide ion water for the production of beverages and foods which not only enhances the storability and antiseptic properties of said beverages and foods and improves their taste but also enables the human body to ingest the required calcium, potassium, magnesium, silicon etc.

According to the present invention there is provided ion water for use in the production of beverages and foods obtainable by the process of acidifying sea water then adding a strong alkali agent to make the pH high, removing any precipitate (a) formed in the sea water, concentrating the remaining sea water and then cooling it, extracting precipitate (b) formed as the sea water cools, and dissolving said precipitate (b) in water to produce said ion water.

Preferably, the ion water comprises an activated calcium material substantially comprising calcium phosphate which has been obtained by the calcination of animal bones at high temperature followed by grinding of the calcined bones.

Preferably also, the ion water comprises a solution of a solid (c) obtained by removal of the water content from the sea water after extraction of the precipitate (b) therefrom.

## DETAILED DESCRIPTION OF PREFERRED EMBODIMENTS

A method for separating the afore-mentioned precipitate (b) and the solid (c) from sea water will now be described in detail.

First, sea water is adjusted to a low pH with strong acid containing sulphate ions.

As the strong acid containing sulphate ions, dilute sulphuric acid of e.g. several % may be used, but it is also possible to use an aqueous solution obtained by adding 3 - 5% of conc. sulphuric acid to an aqueous solution containing activated calcium phosphate dissolved therein and removing a precipitate (hereinafter referred to as P-S acid). This P-S acid exhibits strong acidity of about pH 2.0, but in contrast to violent chemicals such as sulphuric acid, it does not harm the skin and can be used as a highly safe acid. By adding dil. sulphuric acid or P-S acid to sea water in an amount of several % and leaving for 2 - 3 hours, the pH of sea water may be adjusted to as low as pH 2 or below. At this time, there is almost no precipitate produced, but slight precipitates if formed may be removed together with suspended matters present in the starting sea water by means such as filtration.

Thereafter, a strong alkali agent is added to the low pH-adjusted sea water to make the pH high. That is, the sea water once adjusted to a low pH is neutralized and further brought to a high pH, causing salts, e.g. sulphates etc. of the alkaline earth metals and other metals which have lower solubility in high pH ranges to precipitate out. The strong alkali agent can be sodium hydroxide or sodium hydroxide added to an aqueous solution of calcium oxide (hereinafter referred to as Ca-Na aqueous solution) may be used.

The amount of the strong alkali agent required is the amount which can achieve the above-described object or more, and in general, 3% based on sea water in the case of sodium hydroxide (solid) or about 5% in the case of Ca-Na aqueous solution is added and left to stand for 10 hours or longer. By this step, sea water exhibits basically a pH 13 or higher, and a precipitate (a) is formed. This precipitate (a) is removed by filtration etc., and the remaining sea water is heated to evaporate the water content to produce a concentrate. This concentrate is cooled to induce a precipitate (b), which can be separated out by filtration etc. The degree of concentration is such as 20 vol.% or less, preferably about 10 -15% vol.%. based on sea water before concentration.

The thus obtained precipitate (b) has been revealed via elementary analysis to be an alkaline substance mainly containing Na, Mg, K and Ca with a considerable amount of Si, an example of which is shown in Table 1 below, and exhibits a pH of 13.5 or so when dissolved at 10% in water.

## Table 1

Units (mg/kg)

| | | | | | |
|-----|---------|-----|-------|-----|------|
| Ca | 2030 | Al | 33.5 | Mn | 1.39 |
| K | 4470 | B | 169 | Si | 697 |
| Mg | 6.10(%) | Cr | 2.78 | Sr | 194 |
| Na | 33.7(%) | Cu | 5.66 | Zn | 2.09 |
| S | 3.81(%) | Fe | 17.8 | Li | 8.93 |

A solid (c) may be obtained by removing the water content from the filtrate remaining after the removal of the precipitate (b). The removal of the water content is desirably effected by heating to evaporate it under reduced pressure. The thus obtained solid (c) contains the elements set forth in Table 2, mainly comprising salts of sodium ($NaCl$, $Na_2SO_4$, $NaHSO_3$ etc.), hydroxide, oxide thereof etc., and is a strongly alkaline substance exhibiting pH 14 or higher when dissolved in water.

## Table 2

Unit (wt%)

| | | | |
|-----|-------|-----|-------|
| Na | 46.2 | Al | 0.08 |
| K | 1.2 | Ti | 0.012 |
| B | 0.015 | Br | 0.20 |
| Si | 0.48 | Cl | 26 |
| S | 2.5 | | |

A first ion water for the production of beverages and foods according to the present invention may be obtained by dissolving this precipitate (b) in water. The amount to be dissolved in this case varies depending on the beverage and food to be applied, but in general, a stock solution is prepared by dissolving, for example, about 100g in 1 litre of water to make the pH about 13.5, and this stock solution is used by diluting according to the application. When used for the production of noodles, ion water obtained by diluting this stock solution about 20 times to pH about 10 is used. In the case of stored water for whisky-and-water, it is diluted about 100 times or so and used as stored water or water for whisky-and-water as such.

A second ion water according to the present invention may be obtained by dissolving the precipitate (b) and the solid (c) in water at a ratio of at least 1:1 by weight respectively.. The solid (c) herein used is that playing a role as a pH adjusting agent rather than an ion source in the ion water of the present invention. That is, calcium ion water obtained by dissolving the above-described precipitate (b) in water (Japanese patent Application No. 84534/1989) or calcium ion water obtained by dissolving activated calcium phosphate in water (Japanese Patent Publication No. 61079/1985) contains ions useful for the body such as Ca, K, Mg, Si etc.,, but it sometimes happens that the pH of about 13.5 at the time of preparation decreases with time. Therefore, there is no problem when it is used as potable water, but if used as ion water for which a predetermined pH is required, for example, for the production of noodles, the purification of oils, the pH adjustment of foods etc., then pH stability is required. By using the precipitate (b) and the solid (c) in combination, ion water with excellent pH stability may be obtained.

Although the proportion and amounts of the precipitate (b) and the solid (c) used may vary dependent on the application of the ion water, their relative proportions is usually between 5:5 and 10:1 by weight respectively. Further, the amount used is such that, for example, when used as a pH-adjusting agent or for the purification of oils, a stock solution of pH 13 - 14 having a total content of the precipitate (b) and the solid (c) of 10% can be prepared beforehand and then used as required. When used for the production of noodles, ion water with a pH of about 10 and obtained by diluting the stock solution about 20 times is used. Further, in the case of stored water or water for whisky-and-water, it is diluted about 100 times and used as either stored water or water for whisky-and-water.

Also, the ion water can be diluted appropriately and then may be mixed with flour or buckwheat flour in place of salt or salt water to produce noodles, such as wheat vermicelli, spaghetti, chinese noodles etc. The thus prepared noodles are stronger and have improved taste and mouthfeel than conventional products and moreover do not as easily go bad as compared with those where salt or salt water has been used.

The ion water may also be used as water for the production of bread. In this case also, the taste is better and it is also possible to produce salt-reduced bread by reducing the amount of salt used.

Further, the ion water may be used as stored water for emergencies without fear of it going bad for a long time even without special preserving means such as canning etc. Furthermore, as the water contains potassium, magnesium, calcium, silicon etc. and therefore, it becomes very 'tasty water'.

The ion water may also be employed for addition to seasoning agents, for whisky-and-water etc.

Accordingly, the beverages and foods prepared by using this ion water have increased contents of potassium, magnesium and silicon.

Especially as described hereinbelow, by using it in combination with a calcium ion material, the metabolism of calcium, potassium, magnesium etc. in vivo may be enhanced.

Further, the ion water enhances the resistance to decomposition of the beverages and foods to which it has been added by its appropriate pH and the effect of the contained ions, thereby improving their preservation properties.

A third form of ion water obtained by adding a calcium material to the above-described ion water will be described below.

Such an ion water (3) may be obtained by dissolving a specified calcium material and the precipitate (b) obtained from the above-described sea water In water This ion water (3) may further contain the solid (c).

The calcium material may be obtained by the following production process. Bones of e.g. cow, pigs, sheep etc. are calcined at high temperatures to remove the flesh and fat and are thereafter chopped to make bone pieces. These bone pieces are burned at 100° C or higher for 40-50 minutes and are then ground to about 125 μm (120 mesh) as a standard to obtain a finished product.

The ingredients of this finished product are variable but are usually more or less the same as shown in Table 3. All the values are by weight in 100g of the sample.

## Table 3

| | | |
|---|---|---|
| Phosphorus | 17.89 | g |
| Calcium | 40.28 | g |
| Magnesium | 679.2 | mg |
| Potassium | 14 | mg |
| Iron | 0.34 | mg |
| Sodium | 660 | mg |

Such a calcium material can produce a calcium ion water of pH about 13 by dissolving it in water to saturation.

Ion water obtained by dissolving such a calcium material and the precipitate (b) and the solid (c), may be obtained by adding 5-10% of the powdered calcium material to the first or second ion water as described above and dissolving, or appropriately mixing a saturated solution of the calcium material therein. Where the powdered calcium material is dissolved directly, it has been confirmed that it dissolves better as compared with the case where the powdered calcium material is dissolved in ordinary water. It may be applied to various beverages and foods by appropriately diluting as needed. It is needless to say that the necessary amounts of the calcium material, the precipitate (b) and the solid (c) may be dissolved in predetermined amounts of water respectively according to the application.

The mixing ratio and the degrees of dilution of the calcium material (or its aqueous solution) to the precipitate (b) and the solid (c) (or their aqueous solution) are appropriately selected depending on the food applied.

As is clear from the above description, it is possible to provide ion water containing elements believed to be good for the body according to the present invention. In particular, the ion water of the present invention contains such elements as potassium, magnesium etc. and a considerable amount of silicon, and these elements are easily ingested in the body. Further, by using the ion water of the present invention to produce beverages and foods, beverages and foods with excellent storability and having a good taste may be produced by the effect of the above-described elements being present.

The ion water of the present invention may be prepared by using the solid powder according to the use, it may be easily transported and prepared, and thus has a high commercial value. Further, even when stored for a prolonged period of time, the fluctuation in pH is extremely low, and thus it is suitable for uses requiring a predetermined pH.

The above-described ion water may further contain other basic calcium materials for the purpose of adjusting the pH. Such calcium materials include that obtained by calcining a natural calcium material mainly comprising calcium carbonate, such as shells of e.g. scallops etc. at high temperatures such at 1000°C or higher, and thereafter grinding. Such calcium materials are extremely inexpensive because they may be obtained by calcining a starting material of shells which are abundantly obtainable free of cost as industrial waste. This material exhibits a high pH value when dissolved in water. However it has a drawback when added to beverages or foods because it produces a bitter, puckery taste and odour and, further also has the drawback that for example when added to noodles etc., it causes discolouration.

Therefore, when used for noodles, stored water, soya sauce etc., it is desirable that the amount added is restricted to no more than 10% of the total calcium material used. When used for purification of oils, such as lard, fish oils etc., about 20 -30% may be added. Such ion water may be especially utilized as a pH adjusting agent for foods.

EXAMPLES

Ten litres of P-S acid was added to 500 ml of sea water, then left to stand for 3 hours, and thereafter the insoluble matter was removed by filtration. By this process, the pH of the sea water became 1.6. Thereafter, 15 kg of sodium hydroxide was added to 500 l of the pH-lowered sea water, and left to stand for 10 hours. At this time, 10 litres of the sea water remaining after the filtration of the formed insoluble matter was heated to remove the water content to obtain 1.5 l of a concentrated solution. This concentrated solution was rapidly cooled to cause a precipitate, and dried to obtain 200 g of a solid (b).

On the other hand, the filtrate remaining after the removal of the precipitate was heated under reduced pressure to obtain 300 g of a solid (c).

100 g of this solid (b) was dissolved in 1 litre of water to obtain a stock solution of the first ion water. The results of the elementary analysis of this ion water stock solution are shown in Table 4.

50 g of the above-described solid (b) and 50 g of the solid (c) were dissolved in one litre of water to obtain a stock solution of the second ion water. These ion water stock solutions have been confirmed as safe for use in beverages and foods by analysis done by the Japanese Pharmaceutists' Centre of Chiba Prefecture.

## Table 4

| Element | (ug/g) |
|---|---|
| Si | 24 |
| Ca | 6.7 |
| Fe | 0.03 |
| Mg | 0.20 |
| Na | 36000 |
| K | 200 |

On the other hand, 25 kg of a calcium material which was a calcined product of bones and 0.5 kg of a calcium material which was a calcined product of shells were dissolved in 10 l of water to obtain a stock solution of calcium ion water.

Example 1

A mixture of the first ion water and the calcium ion water at a ratio of 1:1 was further diluted 20 times to prepare ion water for the production of noodles.

This ion water was added to flour at 35%, and made into noodles by mixing in a mixer in a conventional manner without using salt at all. These boiled noodles were stored at a storage temperature of 4°C for 8 days, and the number of live bacteria examined. As a result, even after 8 days from production, there was no increase in the number of bacteria, thus showing high storability. Further, as the result of the elementary analysis, these noodles contained large amounts of K, Ca and Mg.

Example 2

A stock solution of the second ion water was prepared, and the pH was examined about 30 days and 60 days after the preparation, to ascertain that the pH was unchanged at pH 13.5, which was the same as that at the time of preparation. This stock solution was diluted about 100 times to prepare potable ion water of pH 8.3. This potable ion water was free from any bleaching powder odour characteristic of tap water and was thus "tasty" water. As the result of its drinking test, 50 out of 100 panelists evaluated it as very tasty while the remaining 50 evaluated as tasty. Also when applied to tea, coffee etc., the results were favourable, and further, when prepared into ice, the product was hard to melt as compared with ice prepared from ordinary water, and the density and the transparency were also higher than usual. When this ice was used in whisky etc., it made whisky tasty.

Example 3

Rice was polished and whilst still retaining the heat of polishing, ion water which had been obtained by diluting the ion water stock solution 20 times was sprayed or scattered on the rice in an amount of 5% based on the weight of the rice while the rice was stirred. Then, after leaving to stand for 30 minutes, the rice was dried well and the water content was made the same as before. This rice was boiled without using salt, and rice balls were prepared. The taste and decomposition of these rice balls were then compared with ordinary rice balls. It was found that the ordinary rice balls produced an odour after one day, but those treated with the ion water did not create any odour. Further, as regards the taste, those prepared from the untreated rice showed differences in taste according to the kinds of rice used whereas for those treated with the ion water, the tackiness and the sugary taste were as good as those of first-grade rice regardless of the kind of rice actually used. The results of the analysis of the rice treated with the ion water are shown in Table 5.

## Table 5

| Calcium | 5 | mg/100 g |
|---|---|---|
| Iron | 0.4 | " |
| Sodium | 4 | " |
| Potassium | 88 | " |
| Magnesium | 30 | " |

Example 4

Ion water for the production of noodles was obtained by mixing the second ion water stock solution and the calcium ion water at a ratio of 1:10 and further diluting it 20 times.

This ion water was added to flour at 35%, and made into noodles by mixing on a mixer without using salt. These boiled noodles were left to stand at room temperature and compared with commercial boiled noodles (those prepared by using salt and sodium malate as a preservative). Although the commercial boiled noodles started to decompose 3 days after the preparation, the boiled noodles of the present example using ion water alone did not show any signs of decomposition even 4 days later.

## Claims

1. Ion water for use in the production of beverages and foods obtainable by the process of
   acidifying sea water then adding a strong alkali agent to make the pH high,
   removing any precipitate (a) formed in the sea water,
   concentrating the remaining sea water and then cooling it,
   extracting precipitate (b) formed as the sea water cools, and
   dissolving said precipitate (b) in water to produce said ion water.

2. Ion water as claimed in Claim 1, characterized in that it comprises an activated calcium material substantially comprising calcium phosphate which has been obtained by the calcination of animal bones at high temperature follwed by grinding of the calcined bones.

3. Ion water as claimed in Claim 1 or Claim 2, characterized in that it comprises a solution of a solid (c) obtained by removal of the water content from the sea water after extraction of the precipitate (b) therefrom.

4. Ion water as claimed in Claim 3, characterised in that the precipitate (b) and the solid (c) are dissolved in the water at a ratio of at least 1:1 by weight respectively.

5. A method of producing ion water for use in the production of beverages and foods comprising the steps of
   acidifying sea water then adding a strong alkali agent to make the pH high,
   removing any precipitate (a) formed in the sea water,
   concentrating the remaining sea water and then cooling it,
   extracting precipitate (b) formed as the sea water cools, and
   dissolving said precipitate (b) in water to produce said ion water.

6. A method as claimed in Claim 5, comprising the additional step of dissolving in water along with said precipitate (b) an activated calcium material substantially comprising calcium phosphate which has been obtained by the calcination of animal bones at high temperature follwed by grinding of the calcined bones.

7. A method as claimed in Claim 5 or Claim 6, comprising the additional step of dissolving in water along with said precipitate (b) a solid (c) obtained by removal of the water content from the sea water after extraction of the precipitate (b) therefrom.

8. A method as claimed in Claim 7, wherein the precipitate (b) and the solid (c) are dissolved in water at a ratio of at least 1:1 by weight respectively.

## Patentansprüche

1. Ionisiertes Wasser zur Verwendung bei der Herstellung von Getränken und Nahrungsmitteln, erhältlich durch das folgende Verfahren:
   Ansäuern von Meerwasser und anschließendes Zusetzen eines starken Alkaliwirkstoffs, um den pH hoch zu machen,
   Entfernen von etwaigem Präzipitat (a), das sich in dem Meerwasser gebildet hat,
   Konzentrieren des verbleibenden Meerwassers und anschließendes Abkühlen desselben,
   Extrahieren von Präzipitat (b), das sich beim Abkühlen des Meerwassers bildet, und
   Lösen des Präzipitats (b) in Wasser, um das ionisierte Wasser zu erzeugen.

2. Ionisiertes Wasser nach Anspruch 1, dadurch gekennzeichnet, daß es ein aktiviertes Calciummaterial aufweist, das im wesentlichen Calciumphosphat aufweist, das durch das Calcinieren von Tierknochen bei hoher Temperatur, gefolgt von Vermahlen der calcinierten Knochen, erhalten worden ist.

3. Ionisiertes Wasser nach Anspruch 1 oder Anspruch 2, dadurch gekennzeichnet, daß es eine Lösung eines Feststoffs (c) aufweist, der durch Entfernen des Wassergehalts aus dem Meerwasser nach Extraktion des Präzipitats (b) daraus erhalten worden ist.

4. Ionisiertes Wasser nach Anspruch 3, dadurch gekennzeichnet, daß das Präzipitat (b) und der Feststoff (c) in dem Wasser in einem Gewichtsverhältnis von wenigstens 1:1 gelöst werden.

5. Verfahren zum Herstellen von ionisiertem Wasser zur Verwendung bei der Herstellung von Getränken und Nahrungsmitteln, das folgende Schritte aufweist:
   Ansäuern von Meerwasser und anschließendes Zusetzen eines starken Alkaliwirkstoffs, um den pH hoch zu machen,
   Entfernen von etwaigem Präzipitat (a), das sich in dem Meerwasser gebildet hat,
   Konzentrieren des verbleibenden Meerwassers und anschließendes Abkühlen desselben,
   Extrahieren von Präzipitat (b), das sich beim Abkühlen des Meerwassers bildet, und
   Lösen des Präzipitats (b) in Wasser, um das ionisierte Wasser zu erzeugen.

6. Verfahren nach Anspruch 5, das folgenden zusätzlichen Schritt aufweist: Lösen eines aktivierten Calciummaterials, das im wesentlichen Calciumphosphat aufweist, das durch das Calcinieren von Tierknochen bei hoher Temperatur, gefolgt von Vermahlen der calcinierten Knochen, erhalten worden ist, zusammen mit dem Präzipitat (b) in Wasser.

7. Verfahren nach Anspruch 5 oder Anspruch 6, das folgenden zusätzlichen Schritt aufweist: Lösen eines Feststoffs (c), der durch Entfernen des Wassergehalts aus dem Meerwasser nach Extraktion des Präzipitats (b) daraus erhalten worden ist, zusammen mit dem Präzipitat (b) in Wasser.

8. Verfahren nach Anspruch 7, wobei das Präzipitat (b) und der Feststoff (c) in Wasser in einem Gewichtsverhältnis von wenigstens 1:1 gelöst werden.

## Revendications

1. Eau additionnée d'ions destinée à être utilisée pour la production de boissons et d'aliments, pouvant être obtenue par le procédé qui consiste à :
   acidifier de l'eau de mer, puis ajouter un agent alcalin fort pour élever le pH,
   éliminer tout précipité (a) formé dans l'eau de mer,
   concentrer l'eau de mer résiduelle et puis la refroidir,
   extraire un précipité (b) formé pendant le refroidissement de l'eau de mer, et
   dissoudre ledit précipité (b) dans de l'eau pour produire ladite eau additionnée d'ions.

2. Eau additionnée d'ions, telle que définie dans la revendication 1, caractérisée en ce qu'elle contient une substance calcique activée constituée sensiblement de phosphate de calcium obtenu par la calcination d'os d'animaux à une température élevée, suivie par un broyage des os calcinés.

3. Eau additionnée d'ions, telle que définie dans la revendication 1 ou la revendication 2, caractérisée en ce qu'elle contient une solution d'une matière solide (c) obtenue par élimination de la teneur en eau de l'eau de mer après extraction du précipité (b) de celle-ci.

4. Eau additionnée d'ions, telle que définie dans la revendication 3, caractérisée en ce que le précipité (b) et la matière solide (c) sont respectivement dissous dans l'eau dans une proportion d'au moins 1:1 en poids.

5. Méthode pour produire de l'eau additionnée d'ions destinée à être utilisée pour la production de boissons et d'aliments, comportant les étapes qui consistent à :
   acidifier de l'eau de mer, puis ajouter un agent alcalin fort pour élever le pH,
   éliminer tout précipité (a) formé dans l'eau de mer,
   concentrer l'eau de mer résiduelle et puis la refroidir,
   extraire un précipité (b) formé pendant le refroidissement de l'eau de mer, et
   dissoudre ledit précipité (b) dans de l'eau pour produire ladite eau additionnée d'ions.

6. Méthode telle que définie dans la revendication 5, comportant l'étape supplémentaire qui consiste à dissoudre dans de l'eau, conjointement avec ledit précipité (b), une substance calcique activée constituée sensiblement de phosphate de calcium obtenu par la calcination d'os d'animaux à une température élevée, suivie par un broyage des os calcinés.

7. Méthode telle que définie dans la revendication 5 ou la revendication 6, comportant l'étape supplémentaire qui consiste à dissoudre dans de l'eau, conjointement avec ledit précipité (b), une matière solide (c) obtenue par l'élimination de la teneur en eau de l'eau de mer après extraction du précipité (b) de celle-ci.

8. Méthode telle que définie dans la revendication 7, selon laquelle le précipité (b) et la matière solide (c) sont respectivement dissous dans de l'eau dans une proportion d'au moins 1:1 en poids.